# EUROPÄISCHE PATENTSCHRIFT

(11) **EP 0 355 456 B1**
(45) Veröffentlichungstag und Bekanntmachung des Hinweises auf die Patenterteilung: **04.01.1995**
(21) Anmeldenummer: 89113830.7
(22) Anmeldetag: 27.07.1989
(51) Int. Cl.: A61M 1/32, A61M 1/36, A61M 1/14

(54) **Blutbegasungsvorrichtung**
Gas treatment device for blood
Dispositif pour le traitement du sang avec un gaz

(30) Priorität: 20.08.1988 DE 8810552 U; 10.02.1989 DE 8901513 U
(43) Veröffentlichungstag der Anmeldung: 28.02.1990
(73) Patentinhaber: Hübner, Karl-Alexander, D-76356 Weingarten (DE)
(72) Erfinder: Hübner, Karl-Alexander, D-76356 Weingarten (DE)
(74) Vertreter: Hubbuch, Helmut, Dipl.-Ing

(56) Entgegenhaltungen:
- DE-A- 3 232 716
- FR-A- 2 258 192
- US-A- 4 245 081

## Beschreibung

Die Erfindung betrifft eine Blutbegasungsvorrichtung mit einem Begasungsbehälter gemäß den Merkmalen des Oberbegriffs von Anspruch 1.

Derartige Blutbegasungsvorrichtungen werden vor allem für die Ozonisierung von Blut im Rahmen der Eigenblutbehandlung eingesetzt. Dabei wird das Blut in den zuvor evakuierten Begasungsbehälter geleitet, darin begast und wieder infundiert. Der Einfachheit halber wird im folgenden nur auf die Begasung mit Ozon Bezug genommen. Selbstverständlich eignet sich die erfindungsgemäße Vorrichtung auch für andere Blutbegasungsverfahren.

Bisher wurden hierzu die üblichen Infusionsflaschen aus Glas verwendet. Sie haben einen mit einem Verschlußstopfen verschlossenen Flaschenstutzen aus Silikongummi.

Der Anschluß an den Patienten erfolgt dabei mit Hilfe eines in den Silikongummistopfen eingeführten Infusionsbesteckes. Der Anschluß an die Gasquelle, üblicherweise ein Ozonisierungsgerät, wird mit Hilfe einer sogenannten Heidelberger Verlängerung hergestellt, die ebenfalls in den Silikonstopfen eingesteckt wird; hinzu kommt ein Gasablaßschlauch und gegebenenfalls eine weitere Kanüle zur Zuführung notwendiger Arzneimittel, insbesondere Gerinnungshemmer.

Mit dieser bekannten Konstruktion sind erhebliche Handhabungsnachteile verbunden. Insbesondere müssen die verschiedenen Anschlußelemente sehr präzise in den Stopfen eingesteckt werden. Übliche Flaschen haben nur einen Stopfendurchmesser von etwa 3 cm. Das Infusionsbesteck allein mißt etwa 2 cm im Durchmesser. Dabei ist noch zu bedenken, dass der Gasanschluß üblicherweise in ein bis in die Nähe des Flaschenbodens ragendes Begasungsrohr übergeht. Wenn aufgrund der engen Platzverhältnisse dieses Rohr beschädigt wird, kann Blut in der Ansaugphase in das Begasungsgerät gelangen und dort erheblichen Schaden anrichten.

Durch die US-PS 4,254,081 ist eine Blutbegasungsvorrichtung bekannt, die am unteren Ende des Behälterkörpers getrennt voneinander den Blutschlauchanschluß und einen unteren Gasschlauchanschluß aufweist, wobei der untere Gasschlauchanschluß ein hydrophobes Filterelement enthält, das für Blut undurchlässig, für Gas jedoch durchlässig ist.

Durch die DE 32 32 716 A1 ist eine Vorrichtung zur Sauerstoffanreicherung von Blut bekannt, die am unteren Ende eines Behälters einen Blutschlauchanschluß und am oberen Ende des Behälters einen Gasschlauchanschluß aufweist. Dieser Gasschlauchanschluß ist zum Evakuieren des Behälters als auch zum Zuführen von Gas zur hyperbaren Gasanreicherung bestimmt.

Ausgehend von der bekannten Blutbegasungseinrichtung liegt der vorliegenden Erfindung die Aufgabe zugrunde, diese so weiterzuentwickeln, dass die Blutbegasungseinrichtung für alle Begasungsmethoden verwendbar ist.

Diese Aufgabe wird durch die Merkmale des Anspruchs 1 gelöst. Vorteilhafte Weiterbildungen sind Gegenstand der Unteransprüche.

Die erfindungsgemässe Blutbegasungseinrichtung zeichnet sich dadurch aus, dass ein zweiter Gasschlauchanschluß am oberen Ende des Behälterkörpers vorgesehen ist. Der Behälterkörper der erfindungsgemässen Blutbegasungsvorrichtung besteht aus Kunststoff. Desweiteren zeichnet sich die Blutbegasungsvorrichtung dadurch aus, dass sich der Blutschlauchanschluß zum Inneren des Behälterkörpers hin trichterförmig erweitert, wobei der Trichter Radialstege zur Abstützung eines Filtersiebs aufweist.

Die einzelnen Maßnahmen des erfindungsgemäßen Vorschlages stehen in engem Zusammenhang zueinander. Zum einen werden durch die Trennung des Blutschlauchanschlusses und des unteren Begasungsanschlusses - zweckmäßigerweise in Form von zwei getrennten Anschlußstutzen - die erwähnten Handhabungsrisiken vermieden. Zum zweiten ist wesentlich, daß sich die Mündung des Blutgasanschlusses im unteren Teil des Behälterkörpers in einer Position befindet, die unterhalb des Füllstandes des Blutes während der Begasung liegt. Dies wird möglich durch die Verwendung des hydrophoben Filterelementes.

Für andere Anwendungszwecke sind bereits Blutbehälter mit zahlreichen verschiedenen Anordnungen von Schlauchanschlüssen bekannt geworden. Beispiele sind der US-PS 4 443 220, der DDR-PS 202 391, der DE-OS 29 20 283 und der französischen Patentanmeldung mit der Publikations-Nr. 2 258 192 zu entnehmen. Keine dieser Druckschriften betrifft jedoch eine Blutbegasungsvorrichtung, so daß ihnen keine Anregung in Richtung auf die vorliegende Erfindung zu entnehmen ist. Außerdem wird in keiner dieser Druckschriften ein hydrophobes Filterelement verwendet.

Die Erfindung ermöglicht eine gegenüber dem Vorbekannten grundsätzlich abweichende Funktionsweise mit vereinfachter Handhabung. Die vorbekannten Infusionsflaschen wurden nämlich zur Blutentnahme mit dem Stopfen nach unten aufgehängt. Die Begasung erfolgte dagegen im umgedrehten Zustand, also mit Stopfen nach oben, wobei nunmehr das erwähnte Begasungsrohr unterhalb des Flüssigkeitsspiegels des Blutes endete, so daß das Gas durch das Blut strömte. Zur Reinfusion mußte die Flasche erneut mit dem Stopfen nach unten aufgehängt werden.

Bei der erfindungsgemäßen Lösung kann der Begasungsbehälter während der Begasung in der gleichen Position - zweckmäßigerweise aufgehängt an einem Infusionsgestell - bleiben, so daß mehrere Handhabungsschritte entfallen.

Der Behälterkörper besteht aus Kunststoff. Dabei ist es besonders vorteilhaft, wenn der Blutschlauch und der obere Gasschlauch permanent mit den entsprechenden Anschlüssen - vorzugsweise durch Kleben - verbunden sind. Begasungsbehälter und Blutschlauch bilden dabei einen gebrauchsfertigen Einwegartikel, der zweckmäßigerweise bereits werkseitig evakuiert ist.

Die Erfindung wird im folgenden anhand eines in den Figuren schematisch dargestellten Ausführungsbeispiels näher erläutert; es zeigen:
- Fig. 1: eine erfindungsgemäße Blutbegasungsvorrichtung,
- Fig. 2: einen Längsschnitt durch einen Begasungsbehälter für eine Vorrichtung nach Fig. 1,
- Fig. 3: einen Ausschnitt III aus Fig. 1 sowie das Ende eines Gasschlauches mit Einsteckkanüle für das Impfventil,
- Fig. 4: einen Querschnitt durch ein Impfventil entlang der Linie IV-IV in Fig. 3,
- Fig. 5: eine Aufsicht auf einen Blutschlauchanschluß vom Inneren des Begasungsbehälters her.

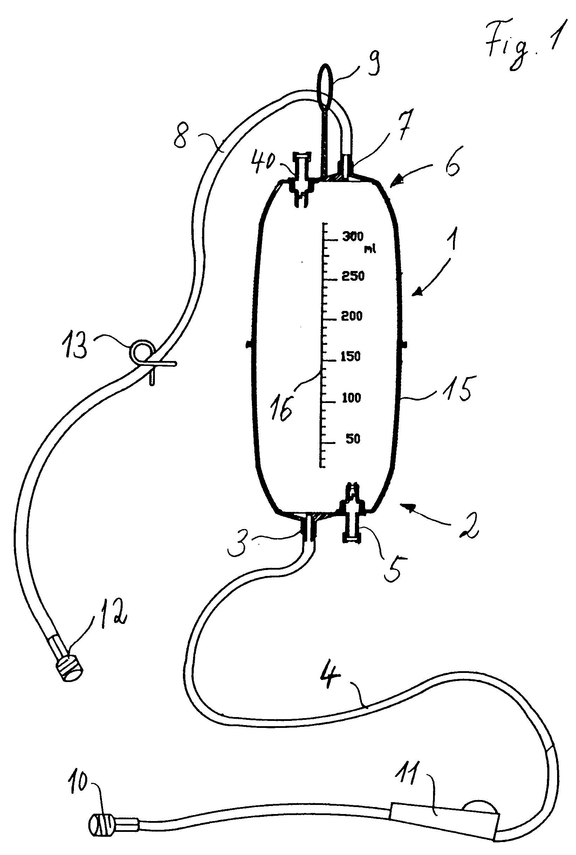

Der Begasungsbehälter 1 der in Fig. 1 dargestellten Blutbegasungsvorrichtung hat an seinem unteren Ende 2 einen Anschluß 3 für einen Blutschlauch 4 und einen Anschluß 5 für einen hier nicht dargestellten unteren Gasschlauch.

Am oberen Ende 6 des Begasungsbehälters 1 befindet sich ein zweiter Gasschlauchanschluß 7, an den ein Gasschlauch 8 angeschlossen ist.

Die Anordnung der Blut- bzw. Gasanschlüsse "am Ende" des Begasungsbehälters 1 ist nicht dahingehend zu verstehen, daß sie von unten bzw. von oben angeschlossen sein müssen. Selbstverständlich ist auch eine Zuführung beispielsweise radial von der Seite her möglich, solange nur gewährleistet ist, daß der untere Gasanschluß 5 sich unterhalb des in der Praxis niedrigsten und der obere Gasanschluß 7 sich oberhalb des in der Praxis höchsten Flüssigkeitsspiegels des Blutes befindet.

Der Begasungsbehälter 1 wird bei Benutzung mit einer zweckmäßigerweise angespritzten Aufhängeöse 9 an einem nicht dargestellten Infusionsgestell aufgehängt. Am Ende des Blutschlauches 4 befindet sich eine geeignete Kupplung 10, beispielsweise ein Luerlock-Anschlußelement zur Verbindung mit einer Infusionsnadel, durch die dem Patienten Blut entnommen werden kann. Die Ansauggeschwindigkeit läßt sich mit Hilfe einer in der Infusionstechnik üblichen Rollklemme 11 dosieren.

Das zum Ansaugen des Blutes erforderliche Vakuum in dem Behälterkörper 1 kann durch Abpumpen über den oberen Gasschlauch 8 erzeugt werden, der zweckmäßigerweise ebenfalls eine Luerlock-Kupplung 12 zum Anschluß an ein entsprechendes Gerät aufweist.

Vorzugsweise wird jedoch die Blutbegasungsvorrichtung bereits werkseitig evakuiert und dem Verwender als gebrauchsfertiger Einmalartikel zur Verfügung gestellt. Zu diesem Zweck wird für den Blutschlauch 4 eine im geschlossenen Zustand vakuumdichte Rollklemme 11 verwendet. Der Gasschlauch 8 ist durch ein vakuumabdichtendes Klemmelement 13 abgesperrt. Die Schläuche 4, 8 sind dabei, beispielsweise durch Kleben, permanent mit den entsprechenden Anschlüssen 3, 7 verbunden. Auch im übrigen ist der Behälterkörper 1 selbstverständlich vakuumdicht.

Der Behälterkörper 15 des Behälters 1 besteht zweckmäßigerweise aus einem durchsichtigen Kunststoffmaterial, insbesondere Acrylglas (Plexiglas) mit einer Skalierung 16, mit deren Hilfe sich die angesaugte Blutmenge leicht kontrollieren läßt.

Nachdem die für die jeweilige Behandlung gewünschte Blutmenge angesaugt ist, erfolgt die Begasung. Dabei werden zwei verschiedene Verfahren unterschieden, die sich beide mit der erfindungsgemäßen Blutbegasungsvorrichtung leicht durchführen lassen.

Bei der sogenannten hyperbaren Ozonisierung wird Ozon unter leicht erhöhtem Druck in den Gasraum oberhalb der Blutoberfläche geleitet. Dies geschieht mit Hilfe des Schlauches 8, der über die Kupplung 12 an ein geeignetes Gerät, beispielsweise das Gerät "Humazon" der Technomed GmbH, Weingarten, Bundesrepublik Deutschland,angeschlossen wird.

Wenn dagegen eine normale Ozonisierung durchgeführt werden soll, bei der man das Gas durch das Blut strömen läßt, wird ein hier nicht dargestellter Gasschlauch an den unteren Gasschlauchanschluß 5 angeschlossen. Das Gas durchströmt das Blut und entweicht durch den oberen Gasschlauch 8. In der Durchgangsöffnung des Gasschlauchanschlusses 5 befindet sich ein hydrophobes Filterelement, welches einerseits das Gas von außen in das Innere des Begasungsbehälters strömen läßt und andererseits für Blut undurchlässig ist.

Derartige hydrophobe Filterelemente werden für andere Anwendungszwecke kommerziell angeboten. Geeignet ist beispielsweise das Produkt 0,2 µm Hydrophob der Firma Schleicher & Schuell, 3354 Dassel, Bundesrepublik Deutschland Die Erfindung bezieht sich jedoch allgemein auf jedes Filterelement, das die genannten Bedingungen (Gasdurchlässigkeit, Blutundurchlässigkeit) erfüllt und physiologisch unbedenklich ist.

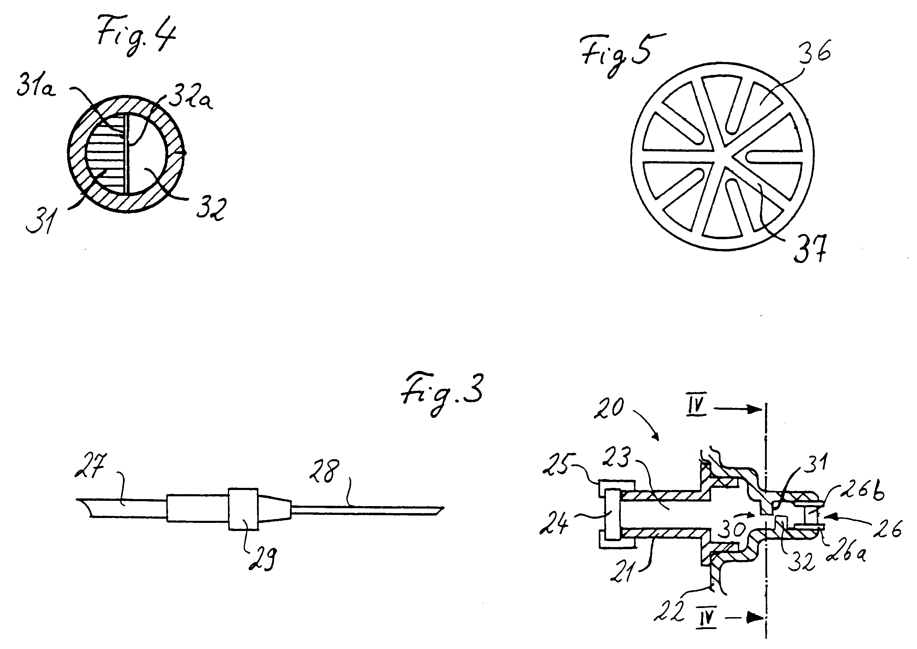

Der Anschluß eines Begasungsschlauches an den Gasschlauchanschluß 5 kann in verschiedener Weise, beispielsweise wiederum mit einer Luerlock-Verbindung erfolgen. Besonders bevorzugt ist jedoch eine Gestaltung als Impfventil, wie sie vergrößert in Fig. 3 dargestellt ist.

Das Impfventil 20 weist einen Rohrstutzen 21 auf, der gasdicht in die Wandung 22 des Behälterkörpers 15 eingesetzt (beispielsweise geklebt) ist. Die Ausnehmung 23 des Rohrstutzens 21 ist nach außen hin durch eine Einstechdichtung in Form einer Dichtungsplatte 24 aus gummielastischem Material verschlossen, die dicht mit dem Rohrstutzen 21, beispielsweise - wie dargestellt - mit Hilfe eines Klemmringes 25 verbunden ist.

Zum Inneren des Behälters 1 hin ist die hier durch eine nach innen gewölbte Ausformung der Wandung 22 gebildete Ausnehmung 23 durch das hydrophobe Filterelement 26 abgeschlossen. Es besteht im dargestellten Fall aus einer Kunststoffhülse 26a und einem darin eingefaßten hydrophoben Filter 26b, der die erwähnten Durchlässigkeitseigenschaften aufweist. Zur Abdichtung zwischen dem hydrophoben Filterelement 26 und der Innenwand des Stutzens 21 genügt eine Pressdichtung, jedoch kann auch hier sicherheitshalber eine Klebeverbindung gewählt werden.

In Figur 3 ebenfalls schematisch dargestellt ist das Ende eines unteren Gasschlauches 27 mit einer Einstechkanüle 28. Die Verbindung zwischen dem Gasschlauch 27 und dem Behälter läßt sich einfach dadurch herstellen, daß die Kanüle 28 in die Einstechdichtung 24 eingestochen wird.

Dabei ist von besonderer Bedeutung, daß zuverlässig verhindert wird, daß die spitze Kanüle 28 das hydrophobe Filterelement 26 beschädigt und dadurch Blut in das Ozonisierungsgerät gelangen kann. Zu diesem Zweck weist das Impfventil 20 eine Durchstechsperre 30 auf. Sie kann beispielsweise einfach darin bestehen, daß der Stutzen 21 länger ist als die Kanüle 28, so daß deren Kopfstück 29, welcher selbstverständlich größer sein muß als der Durchmesser der Ausnehmung 23, gegen den Stutzen 21 anschlägt und dadurch die Einstechtiefe begrenzt wird. Dieses Prinzip funktioniert jedoch nur, wenn die Länge des Stutzens auf die Länge der verwendeten Kanülen 28 abgestimmt ist.

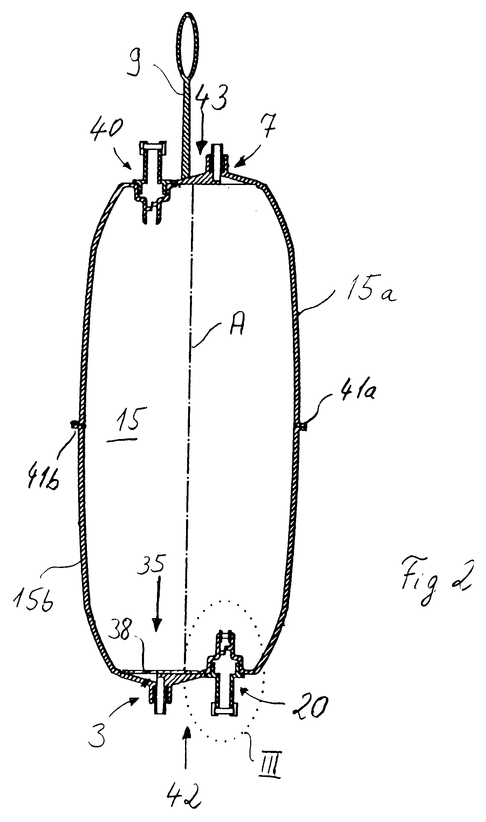

Bevorzugt ist eine Durchstechsperre 30 mit mindestens zwei in die Durchlaßöffnung ragenden Sperrelementen 31, 32, die, wie aus den Figuren 2 und 3 zu ersehen ist, gegeneinander axial versetzt sind. Aus spritztechnischen Gründen ist es vorteilhaft, wenn die Vorderkanten 31a und 32a (Fig. 4) der Sperrelemente 31, 32 in der gleichen, zur Achse des Stutzens 21 parallelen, Ebene liegen.

Figur 5 zeigt in Aufsicht aus Richtung des Pfeiles 35 die Mündung des Blutschlauchanschlusses 3. Dieser erweitert sich zum Behälterinnern hin in Form eines Trichters 36. Radial angeordnete Stege 37 dienen als Abstützung eines Filtersiebes 38 (Fig. 2), das an seinem Rand durch Schweißen oder Kleben mit dem Behälterkörper 15 verbunden ist.

Vorteilhafterweise besteht der Behälterkörper 15 aus zwei identischen Teilen 15a und 15b. Der obere Gasschlauchanschluß 7 ist identisch mit dem Blutschlauchanschluß 3 ausgebildet, wobei jedoch das Filtersieb 38 nicht notwendig ist und deswegen weggelassen werden kann. Zweckmäßigerweise ist dabei am oberen Ende des Behälters 1 ein zweites Impfventil 40 vorgesehen, welches ebenso wie das erste Impfventil 20 gestaltet ist, aber kein hydrophobes Filterelement aufweist.

Es kann beispielsweise verwendet werden, um Arzneimittel, insbesondere Gerinnungshemmer, mit Hilfe einer Spritze zuzuführen.

Um zwei identische Teile 15a und 15b leicht und zuverlässig miteinander verkleben oder verschweißen zu können, ist zweckmäßigerweise der Verbindungsflansch 41a, 41b in besonderer Weise gestaltet. Seine Profilierung ist zweigeteilt, wobei eine Hälfte des Umfanges ein hervorspringendes Profil und die andere Hälfte den gleichen Durchmesserbereich in entsprechendes zurückspringendes Profil aufweist.

Auch die in Figur 2 dargestellte Gehäuseform hat sich als zweckmäßig erwiesen. Günstig ist es, wenn bei der dargestellten im wesentlichen zylindrischen Form des Behälterkörpers 15 dessen vertikal zur Achse A verlaufenden Stirnflächen 42, 43 verhältnismäßig klein sind und allmählich mit einer abgerundeten Formgebung auf den vollen Behälterdurchmesser übergehen. In ihrer allgemeinen Form ist die Erfindung jedoch nicht auf die dargestellte im wesentlichen zylindrische Formgebung beschränkt.

## Patentansprüche

1. Blutbegasungsvorrichtung mit einem Begasungsbehälter (1), dessen Behälterkörper (15) einen Anschluß zum flüssigkeits- und gasdichten Anschließen eines Blutschlauches und mindestens eines Gasschlauches aufweist, wobei am unteren Ende des Behälterkörpers (15) getrennt voneinander der Blutschlauchanschluß (3) und ein unterer Gasschlauchanschluß (5) vorgesehen sind, wobei der untere Gasschlauchanschluß (5) ein hydrophobes Filterelement (26) enthält, das für Blut undurchlässig, für Gas jedoch durchlässig ist,
dadurch gekennzeichnet,
daß ein zweiter Gasschlauchanschluß (7) am oberen Ende des Behälterkörpers (15) vorgesehen ist,
daß der Behälterkörper (15) aus Kunststoff besteht, und
daß sich der Blutschlauchanschluß (3) zum Innern des Behälterkörpers hin trichterförmig erweitert, wobei der Trichter Radialstege (37) zur Abstützung eines Filtersiebes (38) aufweist.

2. Blutbegasungsvorrichtung nach Anspruch 1, dadurch gekennzeichnet, daß ein Blutschlauch (4) permanent mit dem Blutschlauchanschluß (3) und ein oberer Gasschlauch (8) permanent mit dem oberen Gasschlauchanschluß (7) verbunden ist.

3. Blutbegasungsvorrichtung nach Anspruch 2, dadurch gekennzeichnet, daß der obere Gasschlauch (8) ein vakuumdicht abdichtendes Klemmelement (13) aufweist.

4. Blutbegasungsvorrichtung nach Anspruch 1, dadurch gekennzeichnet, daß der untere Gasschlauchanschluß (5) als Impfventil (20) mit einer Einstechdichtung (24) aus gummielastischem Material ausgebildet ist.

5. Blutbegasungsvorrichtung nach Anspruch 4, dadurch gekennzeichnet, daß das Impfventil (20) eine Durchstechsperre (30) aufweist.

6. Blutbegasungsvorrichtung nach Anspruch 5, dadurch gekennnzeichnet, daß die Durchstechsperre (30) mindestens zwei in die Durchlaßöffnung (23) ragende Sperrelemente (31, 32) aufweist, die axial gegeneinander versetzt sind.

7. Blutbegasungsvorrichtung nach Anspruch 4, dadurch gekennzeichnet, daß am oberen Ende des Behälterkörpers (15) ein zweites Impfventil (40) vorgesehen ist.

8. Blutbegasungsvorrichtung nach Anspruch 7, dadurch gekennzeichnet, daß der Behälterkörper (15) aus zwei identischen Teilen (15a, 15b) besteht.

## Claims

1. A blood aeration device including an aeration container (1), the body (15) of which includes a connection for fluid- and gas-tight connection of a blood tube and at least one gas tube, with the bottom end of the container body (15) including a separate blood tube connection (3) and a lower gas tube connection (5), with the lower gas tube connection (5) including a hydrophobic filter element (26) being impermeable to blood but permeable to gas,
characterized in that,
a second gas tube connection (7) is provided at the top end of container body (15),
the container body (15) is made from plastic and
the blood tube connector (3) expands in funnel shape towards the interior of the container body, with the funnel including radial webs (37) to support a filter screen (38).

2. A blood aeration device according to claim 1, characterized in that a blood tube (4) is permanently connected to a blood tube connection (3) and a top gas tube (8) is permanently connected to the top gas tube connection (7).

3. A blood aeration device according to claim 2, characterized in that the top gas tube (8) includes a vacuum-tight sealing and clamping unit (13).

4. A blood aeration device according to claim 1, characterized in that the lower gas tube connection (5) forms an injection valve (20) including an infeed seal (24) made of elastic rubber material.

5. A blood aeration device according to claim 4, characterized in that the injection valve (20) includes a penetration barrier (30).

6. A blood aeration device according to claim 5, characterized in that the penetration barrier (30) includes at least two barrier units (31, 32) projecting into the inlet (23), being axially offset against one another.

7. A blood aeration device according to claim 4, characterized in that a second vaccination valve (40) is provided at the top end of the container body (15).

8. A blood aeration device according to claim 7, characterized in that the container body (15) consists of two identical parts (15a, 15b).

## Revendications

1. Dispositif pour le traitement du sang avec un gaz comportant un récipient (1) de traitement avec un gaz dont le corps (15) présente un raccord permettant de raccorder de façon étanche au liquide et au gaz un tuyau de sang et au moins un tuyau de gaz, dispositif pour lequel sont prévus, à l'extrémité inférieure du corps (15) du récipient (1), séparés l'un de l'autre, un raccord (3) pour tuyau de sang et un raccord inférieur (5) pour tuyau de gaz, ledit raccord inférieur (5) comportant un élément filtrant (26) hydrophobe, imperméable au sang quoique perméable au gaz, caractérisé
en ce qu'un second raccord (7) pour tuyau de gaz est prévu à l'extrémité supérieure du corps (15) du récipient (1)
en ce que le corps (15) du récipient (1) est en plastique, et
en ce que le raccord (3) pour tuyau de sang s'élargit en entonnoir vers l'intérieur du corps (15) du récipient (1), ledit entonnoir présentant des nervures radiales (37) pour le support d'un filtre (38).

2. Dispositif pour le traitement du sang avec un gaz selon la revendication 1 caractérisé en ce qu'un tuyau de sang (4) est relié de façon permanente au raccord (3) pour tuyau de sang et en ce qu'un tuyau de gaz supérieur (8) est relié de façon permanente au raccord (7) supérieur pour tuyau de gaz.

3. Dispositif pour le traitement du sang avec un gaz selon la revendication 2 caractérisé en ce que le tuyau de gaz supérieur (8) présente un élément de serrage (13) étanche au vide.

4. Dispositif pour le traitement du sang avec un gaz selon la revendication 1 caractérisé en ce que le raccord inférieur (5) pour tuyau de gaz constitue une soupape d'introduction (20) avec un site d'injection (24) en matériau caoutchouteux élastique.

5. Dispositif pour le traitement du sang avec un gaz selon la revendication 4 caractérisé en ce que la soupape d'introduction (20) présente un arrêt en perforation (30).

6. Dispositif pour le traitement du sang avec un gaz selon la revendication 5 caractérisé en ce que l'arrêt en perforation (30) présente au moins deux éléments de barrage (31,32) se dressant dans l'ouverture de passage (23) désaxés l'un par rapport à l'autre.

7. Dispositif pour le traitement du sang avec un gaz selon la revendication 4 caractérisé en ce qu'il est prévu à l'extrémité supérieure du corps (15) du récipient (1) une seconde soupape d' introduction (40).

8. Dispositif pour le traitement du sang avec un gaz selon la revendication 7 caractérisé en ce que le corps (15) du récipient (1) est constitué de deux parties identiques (15a,15b).
